# EUROPÄISCHE PATENTANMELDUNG

(11) **EP 1 595 868 A1**
(43) Veröffentlichungstag der Anmeldung: **16.11.2005**
(21) Anmeldenummer: 05102210.1
(22) Anmeldetag: 21.03.2005
(51) Int. Cl.: C07D 211/94

(54) **Verfahren zur Herstellung von 4-substituierten 2,2,6,6-Tetramethyl-piperidin-N-oxy- und 2,2,6,6-Tetramethyl-piperidin-N-hydroxy-verbindungen**

(30) Priorität: 10.05.2004 DE 102004023640
(71) Anmelder: Degussa AG, 40474 Düsseldorf (DE)
(72) Erfinder: Osterholt, Clemens, 46286, Dorsten (DE); Poll, Heinz-Günter, 45770, Marl (DE); Meyer, Oliver, 48151, Münster (DE); Kübelbäck, Thomas, 48249, Dülmen (DE)

(57) **Zusammenfassung**

Die vorliegende Erfindung betrifft Verfahren zur Herstellung von 4-substituierten 2,2,6,6-Tetramethyl-piperidin-N-oxy-verbindungen [II] und von Gemischen aus [II] und 4-substituierten 2,2,6,6-Tetramethyl-piperidin-N-hydroxy-verbindungen [III] 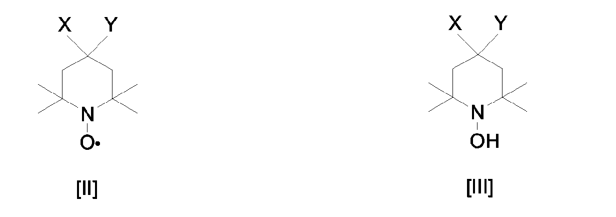 wobei X+Y = O sein kann oder für ein cyclisches Ketal stehen kann mit den Resten 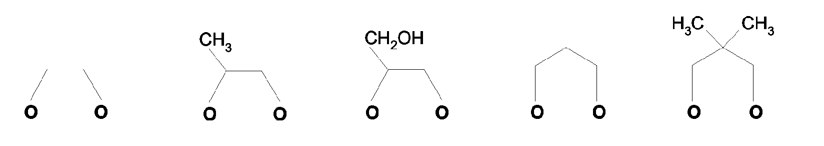 oder für ein offenkettiges Ketal stehen kann, bei dem X= O-R und Y= O-R' bedeuten, wobei R und R' gleich oder verschieden voneinander sein können und jeweils für CH₃, CH₂-CH₃, CH₂-CH₂-CH₃, CH(CH₃)-CH₃, CH₂-CH₂-CH₂-CH₃ und CH₂-CH(CH₃)-CH₃ stehen können,
durch Oxidation von entsprechenden 4-substituierten 2,2,6,6-Tetramethylpiperidinen [I] mit

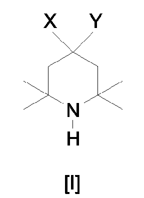

Hilfe von Wasserstoffperoxid in Gegenwart von Alkali- und/oder Ammoniumhydrogencarbonat und ggf. in Gegenwart eines Lösungsmittels, bei dem die Reaktion unter Zusatz von solchen Brönstedtsäuren durchgeführt wird, die eine Säurestärke größer als die des eingesetzten Hydrogencarbonats besitzen.

## Beschreibung

Die Erfindung betrifft ein Verfahren zur Herstellung von 4-substituierten 2,2,6,6-Tetramethyl-piperidin-N-oxy-verbindungen [II] und von 4-substituierten 2,2,6,6-Tetramethyl-piperidin-N-hydroxy-verbindungen [III] durch Oxidation von entsprechenden 4-substituierten 2,2,6,6-Tetramethylpiperidinen [I] mittels Wasserstoffperoxid in Gegenwart von Hydrogencarbonatsalzen als Katalysator, gemäß den folgenden Gleichungen, 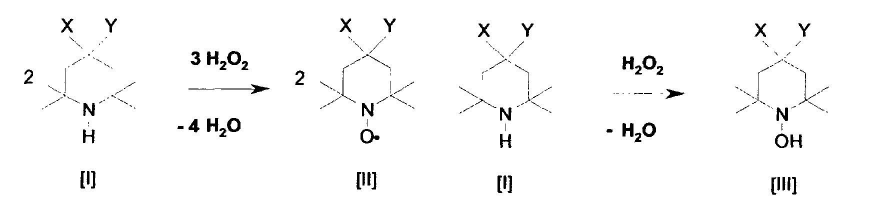 wobei X+Y = O sein kann oder für ein cyclisches Ketal stehen kann mit den Resten 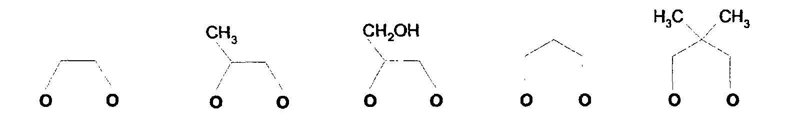 oder für ein offenkettiges Ketal stehen können, bei dem X= O-R und Y= O-R' bedeuten, wobei R und R' gleich oder verschieden voneinander sein können und jeweils für CH₃, CH₂-CH₃, CH₂-CH₂-CH₃, CH(CH₃)-CH₃, CH₂-CH₂-CH₂-CH₃ und CH₂-CH(CH₃)-CH₃ stehen können.

Aufgrund ihrer Stoffeigenschaften finden die häufig auch als TEMPO-Derivate bezeichneten stabilen N-Oxyl-Radikale [II] eine breite Anwendung als Oxidationskatalysatoren, als Polymerisationsinhibitoren oder als Massenregulatoren bei radikalischen Polymerisationen. Insbesondere wird dabei das in der Anmeldung auch beschriebene 2,2,6,6-Tetramethyl-4-oxo-piperidin-N-oxy (kurz 4-Oxo-TEMPO oder TEMPON genannt) [vgl. II mit X+Y = O] zur Stabilisierung von ungesättigten Monomeren eingesetzt.

Zur Oxidation von beispielsweise Triacetonamin (TAA) zu dem entsprechenden stabilen N-Oxyl-Radikal 4-Oxo-TEMPO sind aus der Literatur mehrere Methoden bekannt.
Als Oxidationsmittel für diese Reaktion wurden unter anderem Persulfat (Tetrahedron Lett. 1995, 36, 31, 5519-5522), Persulfonsäure (Bull.Acad.Sci.USSR Div.Chem.Sci (Engl. Transl.) 1990, 39, 1045-1047), Dimethyldioxiran (Tetrahedron Lett. 1988, 29, 37, 4677-4680), Ozon (SU963987) oder organische Hydroperoxide (RU 2139859) eingesetzt. Auch die elektrochemische Oxidation von Triacetonamin findet sich in der Literatur beschrieben (PL148157).

In den weitaus meisten Fällen wird die sekundäre Aminfunktion jedoch mit Hilfe von Wasserstoffperoxid als Oxidationsmittel zur N-Oxylgruppe oxidiert (z. B. Tetrahedron 1992, 48, 9939-9950; Chemical Papers 1988, 42, 2, 243-248; Tetrahedron 1985, 41, 1165-1172; J. Prakt. Chem. 1985, 327, 6, 1011-1014; Chem. Pharm. Bull. 1980, 28, 3178-3183; US 5817824). Im Gegensatz zu den zuvor genannten Oxidationsmitteln hat Wasserstoffperoxid den großen Vorteil, dass als Kopplungsprodukte nur Wasser und gegebenenfalls Sauerstoff entstehen.
Die in der Literatur bisher beschriebenen Oxidationsverfahren von TAA mit Wasserstoffperoxid unterscheiden sich somit hauptsächlich hinsichtlich der dabei zusätzlich eingesetzten Oxidationskatalysatoren.

Am häufigsten wird in der Literatur der Einsatz von Natriumwolframat als Oxidationskatalysator beschrieben (Tetrahedron 1992 48, 9939-9950; Chemical Papers 1988, 42, 2, 243-8; Tetrahedron 1985, 41, 1165-1172; J. Prakt. Chem. 1985, 327, 6, 1011-1014, US 5817824). Nachteil dabei ist neben teilweise unbefriedigenden Ausbeuten unter anderem auch der relativ hohe Preis und die Abwasserbelastung mit Wolframsalzen.
Bei diesen Verfahren werden teilweise z.B. die Alkalisalze von Ethylendiamintetraessigsäure (EDTA) zusätzlich eingesetzt, wie beispielsweise in US 5817824, hauptsächlich wegen ihrer Basenwirkung und weil ihre komplexierenden Eigenschaften gegenüber Schwermetallionen wahrscheinlich zur Stabilität von Wasserstoffperoxid beitragen (US 5817824, Spalte 10, Zeilen 32 bis 36). Trotzdem werden hier nur Ausbeuten von 40,8 bis 58 % d. Th. erzielt.

In der italienischen Patentanmeldung IT 2000MI1052 wird ein Verfahren beschrieben, bei dem Triacetonaminderivate mit Wasserstoffperoxid zu den entsprechenden N-Oxy-Derivaten umgesetzt werden, und bei dem ausschließlich Phosphonsäuren oder deren Salze, wie Diethylentriaminpentamethylenphosphonsäure-Heptanatriumsalz, als Katalysatoren eingesetzt werden. In der betreffenden Umsetzung von Triacetonamin zu 4 Oxo-TEMPO beträgt der Umsatz 93,3 %, die Selektivität jedoch nur 67,6 %, was einer Ausbeute von nur 63 % entspricht (Beispiel 7). Die entsprechenden Ketale werden zwar erwähnt, jedoch keine Beispiele oder Ausbeuten dafür angegeben. Die wasserlöslichen Phosphonsäuren sind außerdem als Verunreinigungen für bestimmte Anwendungen und ggf. auch im Abwasser nicht erwünscht und verteuern aufgrund ihres hohen Preises das Herstellverfahren.

Aber auch der Einsatz von Natriumcarbonat oder Natriumhydrogencarbonat als Katalysator ist beschrieben z.B. in Dokl. Chem. (Engl. Transl.) 1981, 261, 466-467 (entspricht Dokl. Akad. Nauk SSSR Ser. Kim. 1981, 261, 109-110). Diese Methode hat, obwohl sehr einfach, den Nachteil, dass für eine Rohausbeute von 95 % sehr lange Reaktionszeiten von 4 - 5 Tagen nötig sind und relativ hohe Überschüsse von 2,7 Äquivalenten an Wasserstoffperoxid gebraucht werden.

Bei der in J. Prakt. Chem. 1985, 327, 6, 1011-1014 beschriebenen Nacharbeitung des gleichen Verfahrens wurden entsprechend nur noch 73 % d.Th. nach zwei Tagen Reaktionszeit erhalten. Außerdem ist die Methode nur in einem Glasgefäß beschrieben und eignet sich, wie hier gefunden wurde, nicht für den Einsatz in technischen Apparaturen, die üblicherweise in Metall ausgelegt sind.

In US 5629426 wird ein Verfahren speziell zur Herstellung von 4-Hydroxy-2,2,6,6-tetramethylpiperidin-N-oxid durch Oxidation des entsprechenden Amins in Gegenwart von Carbonat oder Bicarbonat und EDTANa₂ als chelatisierendes Agens beschrieben. Dieses dient in erster Linie dazu, Spuren von Eisen oder anderen Metallen aus der Redaktionsmischung während des Herstellungsprozess abzufangen und auf diese Weise der Zerstörung von Wasserstoffperoxid vorzubeugen. Aber auch EDTA ist als Verunreinigung für bestimmte Anwendungen nicht erwünscht. Außerdem verteuert es aufgrund seines hohen Preises ebenfalls das Herstellverfahren. Darüber hinaus wird über die Ausführbarkeit bei den o. g. TAA-Derivaten bzw. in Reaktoren mit Metalloberflächen keine Aussage getroffen. Aufgrund der Beschreibung insbesondere der Größe der Ansätze von < 1 mol Substrat, ist jedoch von Laborbeispielen auszugehen die in Glasgeräten durchgeführt wurden und damit keine verlässliche Aussage über die technische Umsetzbarkeit gestatten.

In EP 574 666 wird lediglich eine Oxidationsmethode für Ketale von Triacetonamin angegeben, die allerdings nur in Glasapparaturen durchgeführt wird und bei der zweiwertige Metallsalze von Erdalkalimetallen und Zink als Katalysatoren Verwendung finden, also ebenfalls eine Methode, die nicht direkt in technischen Metallapparaturen ausführbar ist.

Vor allem im technischen Maßstab wird ein hoher H₂O₂-Verbrauch, sowie ein schlechter Umsatz des zu oxiderenden Substrats jedoch durch mehrere Faktoren hervorgerufen:
So spielen insbesondere mögliche Verunreinigungen wie beispielsweise Schwermetallionen eine bedeutende Rolle bei der Zersetzung von H₂O₂. Ebenso nimmt die Zersetzung mit größerer Reaktoroberfläche, aber auch größerer Rauigkeit der Reaktorfläche zu, da hier permanent Schwermetallspuren aus der Behälterwand abgelöst werden können. Bis zu einem gewissen Grade kann dem durch geeignete Passivierung des Reaktors vor Reaktionsbeginn entgegengewirkt werden. Auch die Rührergeschwindigkeit spielt dabei eine nicht unerhebliche Rolle und eine zu hohe Temperatur kann die Zersetzung von H₂O₂ ebenfalls begünstigen.
Ebenso führt ein hoher pH-Wert, der sich zum Beispiel aus einem zu hohen Anteil von Na₂CO₃ ergeben kann, zu stärkerer H₂O₂-Zerstörung.
So kann auch die Zersetzung von beispielsweise als Katalysator eingesetztem NaHCO₃ gemäß der Gleichung 2 NaHCO₃ -> Na ₂CO₃ + H₂O + CO₂ zu einer verstärkten Bildung von Na₂CO₃ führen mit den genannten nachteiligen Folgen. Hier wurde nun auch gefunden, dass eine verstärkte Zersetzung von Wasserstoffperoxid zu einer vermehrten Bildung von Na₂CO₃ führt, welches über den o. g. pH-Effekt die Zersetzung von Wasserstoffperoxid wiederum beschleunigt.

Es bestand daher die Aufgabe, ein möglichst einfaches, im industriellen Maßstab durchführbares Verfahren zur Herstellung der oben näher bezeichneten 4-substituierten 2,2,6,6-Tetramethyl-piperidin-N-oxy-verbindungen [II] oder von Gemischen aus [II] mit 4-substituierten 2,2,6,6-Tetramethyl-piperidin-N-hydroxy-verbindungen [III] durch Oxidation von entsprechenden 4-substituierten 2,2,6,6-Tetramethylpiperidinen [I] mittels Wasserstoffperoxid bereitzustellen, das die oben genannten Nachteile nicht aufweist und sich problemlos möglichst ohne apparativen Mehraufwand in gängigen technischen Metallapparaturen durchführen lässt.

Insbesondere sollte das Verfahren sich dadurch auszeichnen, dass es mit möglichst niedrigen Überschüssen bzw. Verbräuchen an Reaktanden bei gleichzeitig möglichst kurzen Reaktionszeiten und hohen Raum-Zeit-Ausbeuten auch im industriellen Maßstab durchführbar sein sollte und dabei eine möglichst einfache Form der Aufreinigung möglichst ohne teure oder toxikologisch bedenkliche Hilfsstoffe oder kritische Abfallströme sowie eine möglichst hohe Ausbeute und Reinheit an Zielprodukt ermöglicht.

Gelöst werden diese sowie weitere nicht explizit genannten Aufgaben, die jedoch aus den hierin diskutierten Zusammenhängen ohne weiteres ableitbar oder erschließbar sind durch ein Verfahren nach Anspruch 1. Zweckmäßige Ausformungen und Abwandlungen des erfindungsgemäßen Verfahrens werden in den auf Anspruch 1 rückbezogenen Unteransprüchen unter Schutz gestellt.

Dadurch dass man ein Verfahren zur Herstellung von 4-substituierten 2,2,6,6-Tetramethyl-piperidin-N-oxy-verbindungen [II] oder von Gemischen aus [II] und 4-substituierten 2,2,6,6-Tetramethyl-piperidin-N-hydroxy-verbindungen [III] 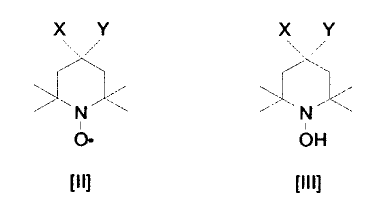 wobei X+Y = O sein kann oder für ein cyclisches Ketal stehen kann mit den Resten 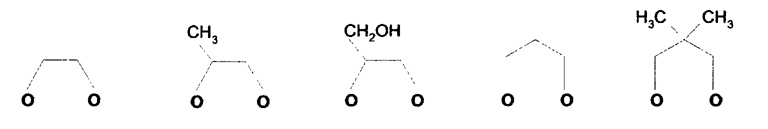 oder für ein offenkettiges Ketal stehen kann, bei dem X= O-R und Y= O-R' bedeuten, wobei R und R' gleich oder verschieden voneinander sein können und jeweils für CH₃, CH₂-CH₃, CH₂-CH₂-CH₃, CH(CH₃)-CH₃, CH₂-CH₂-CH₂-CH₃ und CH₂-CH(CH₃)-CH₃ stehen können,
durch Oxidation von entsprechenden 4-substituierten 2,2,6,6-Tetramethylpiperidinen [I] mit 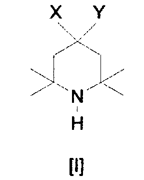 Hilfe von Wasserstoffperoxid in Gegenwart von Alkali- und/oder Ammoniumhydrogencarbonat und ggf. in Gegenwart eines Lösungsmittels, anwendet, bei dem die Reaktion unter Zusatz von solchen Brönstedtsäuren durchgeführt wird, die eine Säurestärke größer als die des eingesetzten Hydrogencarbonats besitzen, gelingt es auf sehr einfache Weise, die 4-substituierten 2,2,6,6-Tetramethyl-piperidin-N-oxy-verbindungen [II] oder Gemische aus [II] mit 4-substituierten 2,2,6,6-Tetramethyl-piperidin-N-hydroxy-verbindungen [III] in hohen Ausbeuten und Selektivitäten bei gleichzeitig niedrigen Verlusten an Wasserstoffperoxid in technischen Apparaturen mit Metalloberflächen ohne apparativen Mehraufwand im industriellen Maßstab herzustellen sowie die weiteren oben näher bezeichneten Nachteile des Standes der Technik zu überwinden.

Gemäß der eingangs angegebenen Reaktionsgleichung können ausgehend von [I] - wie hier gefunden wurde - bei der erfindungsgemäßen Oxidation die analogen N-Hydroxy-Derivate [III] in unterschiedlichen Anteilen mitentstehen. Da die N-Hydroxy-Derivate aber in den meisten Fällen in der gleichen Art und Weise wie die N-Oxy-Derivaten verwendet werden können, weil beide Formen in situ ineinander übergehen können, ist in der Regel keine Trennung notwendig. Das erfindungsgemäße Verfahren bezieht sich daher sowohl auf die Herstellung der reinen N-Oxy-Derivate (II) als auch auf die Herstellung von Gemischen aus II und den entsprechenden N-Hydroxy-Derivaten (III).
Üblicherweise entstehen beim erfindungsgemäßen Verfahren jedoch ≥ 90 Mol % N-Oxy-Derivate und entsprechend ≤ 10 Mol % N-Hydroxy-Derivate, so dass erfindungsgemäß vorzugsweise entweder die nahezu reinen N-Oxy-Derivate bzw. Mischungen mit mindestens 90 Mol % N-Oxy-Derivat erhalten werden.

Unter Brönstedtsäure wird in diesem Zusammenhang jede Säure verstanden, die Protonen abgeben kann. Brönstedtsäuren, die eine Säurestärke größer als die des eingesetzten Hydrogencarbonats besitzen, sind erfindungsgemäß solche, welche einen kleineren pKa-Wert besitzen als Hydrogencarbonat. Somit setzt man beim erfindungsgemäßen Verfahren vorteilhaft mindestens eine Brönstedtsäure ein, die bei einer Temperatur von 25 °C einen pKa-Wert kleiner 10,3 aufweist, vorzugsweise ≤ 9,2, insbesondere -1,5 bis 7,2. Die Ermittlung der pKa-Werte ist an sich bekannt und gängigen Standardwerken der Chemie zu entnehmen.

Als Alkalihydrogencarbonat werden vorzugsweise Lithium-, Natrium, Kalium, Rubidium-und/oder Cäsiumhydrogencarbonat, insbesondere jedoch Natriumhydrogencarbonat eingesetzt. Natriumhydrogencarbonat hat vor allem den Vorteil, dass es sehr preiswert und leicht verfügbar ist.

Hydrogencarbonat dient als Katalysator für die Reaktion und kann daher in unterstöchiometrischen Mengen, vorzugsweise in einer Menge von 0,02 bis 0,5 Moleq, insbesondere jedoch von 0,1 bis 0,25 Moleq Alkali- und/oder Ammoniumhydrogencarbonat bezogen auf das zu oxidierende Amin [I] eingesetzt werden.

Als Brönstedtsäure können sowohl eine als auch mehrere anorganische bzw. organische Säuren oder auch Mischungen von anorganischen und organischen Säuren eingesetzt werden.

Als anorganische Säuren werden dabei vorzugsweise Phosphorsäure, Dihydrogenphosphat und/oder Hydrogenphosphat, insbesondere Alkali- oder Ammoniumdihydrogenphosphat, Dialkali- oder Diammoniumhydrogenphosphat - oder Alkali-/Ammoniumhydrogenphosphat, aber auch Salpetersäure, Schwefelsäure, Alkali- oder Ammoniumhydrogensulfat und/oder Halogenwasserstoffsäuren, und hier besonders bevorzugt Salzsäure, eingesetzt. Alle genannten Säuren haben insbesondere den Vorteil, dass sie allesamt preiswert, leicht verfügbar und im Abwasser in den typischerweise anfallenden Mengen meist unbedenklich sind.

Als organische Säuren werden dabei vorzugsweise Ameisensäure oder eine gesättigte lineare oder ggf. verzweigte ein- oder mehrbasige aliphatische Carbonsäure mit 2 bis 12 C-Atomen verwendet, die mit O-R¹ oder NR²R³ substituiert sein kann, wobei R¹, R² bzw. R³ gleich oder verschieden sein können und Wasserstoff, einen aliphatischen oder cycloaliphatischen, gesättigten Alkylrest mit 1 bis 12 C-Atomen oder eine Carboxyalkylgruppe (CH₂)ₙCOOH mit n = 1 bis 5 bedeuten oder R² und R³ beide zusammen für eine gesättigte, gegebenenfalls alkylsubstituierte Alkylenkette mit 4 bis 11 C-Atomen stehen können, mit der Maßgabe, dass wenn R² und R³ gleichzeitig Wasserstoff oder Alkyl oder beide zusammen Alkylen bedeuten oder wenn R² = Wasserstoff und R³ = Alkyl bedeuten, die mit NR²R³ substituierte Carbonsäure mehrbasig sein muß.
Ganz besonders bevorzugt werden jedoch Essigsäure, Hydroxyessigsäure, Methoxyessigsäure, Propionsäure, Buttersäure, 2-Ethylhexansäure, Oxalsäure, Malonsäure, Bernsteinsäure, Glutarsäure, Zitronensäure, Iminodiessigsäure, Nitrilotriessigsäure oder eine saure Aminosäure eingesetzt. Diese haben insbesondere den Vorteil, dass sie gut verfügbar, überwiegend preiswert und leicht biologisch abbaubar sind.
Unter saurer Aminosäure wird in diesem Zusammenhang jede Aminosäure verstanden, die im Molekül mindestens eine Carboxylgruppe mehr als vorhandene Aminofunktionen aufweist, insbesondere also Asparaginsäure, Glutaminsäure etc..

Als organische Säuren können erfindungsgemäß auch Phosphonsäuren und/oder ihre partiellen Salze, mit der folgenden allgemeinen Formel:

Z(PO₃HₙM₂₋ₙ)ₘ

einzeln oder auch als Gemisch eingesetzt werden, wobei Z ein oder mehrere, lineare oder verzweigte alkylische Radikale oder Diradikale mit insgesamt C₁-C₁₀ darstellt, die insgesamt bis zu 3 Stickstoffatome enthalten können, m Werte von 1 bis 6 und n Werte von 1 oder 2 annehmen kann und M = Alkali oder NH₄ ist.
Die Formel Z(PO₃HₙM₂₋ₙ)ₘ definiert somit eine Gruppe von Phosphonsäuren und ihren partiellen Alkali- oder NH₄ -Salzen, wobei bei den partiellen Alkalisalzen die Natrium- bzw. Kaliumsalze bevorzugt werden. Bei den partiellen Salzen werden bevorzugt jene eingesetzt, bei denen mindestens ein protischer Wasserstoff, der nicht zu einer Ammoniumgruppe oder zu einer ggf. vorhandenen Di- oder Trialkylenammoniumgruppe gehört, im Molekül vorhanden ist, womit sichergestellt wird, dass eine Säure mit ausreichender Säurestärke für das erfindungsgemäße Verfahren vorhanden ist.

Werden Phosphonsäuren und/oder ihre partiellen Salze eingesetzt, so werden dabei bevorzugt: Ethylendiphosphonsäure und ihre partiellen Natrium-, Kalium- und Ammoniumsalze mit der Maßgabe, dass mindestens ein protischer Wasserstoff, der nicht zu einer ggf. vorhandenen Ammoniumgruppe gehört, im Molekül vorhanden ist;
1-Hydroxyethyliden-1,1-Diphosphonsäure und ihre partiellen Natrium-, Kalium- und Ammoniumsalze (HEDP) mit der Maßgabe, dass mindestens ein protischer Wasserstoff, der nicht zu einer ggf. vorhandenen Ammoniumgruppe gehört, im Molekül vorhanden ist;
Amino-trimethylenphosphonsäure und ihre partiellen Natrium-, Kalium- und Ammoniumsalze, mit der Maßgabe, dass mindestens ein protischer Wasserstoff, der nicht zu einer Ammoniumgruppe oder zur Trimethylenammoniumgruppe gehört, im Molekül vorhanden ist; Ethylendiamin-tetramethylenphosphonsäure und ihre partiellen Natrium-, Kalium- und Ammoniumsalze (EDTMP), mit der Maßgabe, dass mindestens ein protischer Wasserstoff, der nicht zu einer Ammoniumgruppe oder zur Trimethylenammoniumgruppe gehört, im Molekül vorhanden ist;
Diethylentriamin-pentamethylenphosphonsäure und ihre partiellen Natrium-, Kalium- und Ammoniumsalze (DTPMP), mit der Maßgabe, dass mindestens ein protischer Wasserstoff, der nicht zu einer Ammoniumgruppe oder zu einer Trialkylenammoniumgruppe gehört, im Molekül vorhanden ist;
Hexamethylendiamin-tetramethylenphosphonsäure und ihre partellen Natrium-, Kalium- und Ammoniumsalze (HMDTMP), mit der Maßgabe, dass mindestens ein protischer Wasserstoff, der nicht zu einer Ammoniumgruppe oder zu einer Trialkylenammoniumgruppe gehört, im Molekül vorhanden ist, entweder einzeln oder als Gemisch eingesetzt.
Die vorstehend genannten Phosphonsäuren haben neben ihrer erfindungsgemäßen Säurewirkung zusätzlich eine stark chelatisierende Wirkung auf Schwermetallionen, sind jedoch relativ teuer.
In der üblichen Ausführungsform werden daher solche Säuren eingesetzt, die keine Phosphonsäuren vom Typ Z(PO₃HₙM₂₋ₙ)ₘ sind.

Als Säuren können grundsätzlich auch chelatisierende organische Säuren wie Ethylendiamintetraessigsäure oder Diethylentriaminpentaessigsäure und jeweils ihre partiellen Natrium-, Kalium- und Ammoniumsalze aber auch Iminodiessigsäure oder Nitrilotriessigsäure und jeweils ihre partiellen Natrium-, Kalium- und Ammoniumsalze bzw. entsprechende Mischungen eingesetzt werden, mit der Maßgabe, dass jeweils mindestens ein protischer Wasserstoff, der nicht zu einer Ammoniumgruppe oder zu einer Di- bzw. Trialkylenammoniumgruppe gehört, im Molekül vorhanden ist. Die vorstehend genannten chelatisierenden organischen Säuren sind jedoch ebenfalls relativ teuer.
In der üblichen Ausführungsform der Erfindung werden daher keine der o.g. chelatisierenden organischen Säuren eingesetzt.

Erfindungsgemäß setzt man vorteilhafterweise insgesamt 0,01 - 0,5 Moleq, insbesondere jedoch 0,03-0,1 Moleq der Brönstedtsäuren bezogen auf das zu oxidierende Amin [I] ein.

Die Reaktion wird dabei vorzugsweise so durchgeführt, dass nach Beendigung der Reaktion die wässrige Phase im Falle eines zweiphasigen Gemisches bzw. die wässrig/organische Phase im Falle eines homogenen Reaktionsmediums (bei Verwendung entsprechender Lösungsmittel) einen pH-Wert von 7,0 bis 10,0 aufweist. Besonders bevorzugt wird die Reaktion jedoch so durchgeführt, dass der pH-Wert nach Beendigung der Reaktion bei 8,0 bis 9,6, insbesondere bei 8,2 bis 9,2 liegt.
Der pH-Wert wird mit einer pH-Elektrode bei Raumtemperatur bestimmt. Der pH-Wert kann hauptsächlich durch geeignete Auswahl der einzusetzenden Brönstedtsäuren bzw. deren Mengen und Konzentrationen beeinflusst werden, aber auch durch geeignete Auswahl des verwendeten Hydrogencarbonats und von dessen Mengen bzw. Konzentration. Geeigneterweise kann der pH-Wert natürlich auch jederzeit während der Reaktion bestimmt werden und in der Folge durch Zugabe geeignete Mengen Säure so nachjustiert werden, dass am Ende der Reaktion der bevorzugte pH-Bereich erreicht wird.

Erfindungsgemäß bevorzugt wird Wasserstoffperoxid in wässriger Lösung mit einer Konzentration im Bereich von 10 bis 90 Gew.%, insbesondere im Bereich von 20 bis 60 Gew.% , jedoch ganz besonders bevorzugt von 30 bis 50 Gew.% verwendet.

Das erfindungsgemäße Verfahren kann sowohl ohne Zusatz von Lösungsmitteln in wässriger Lösung oder Suspension als auch unter bestimmten Umständen unter Zusatz von Lösungsmitteln, wie zum Beispiel Alkohole, Diole, Etherverbindungen, Ketone, aliphatische, cycloaliphatische, aromatische und/oder araliphatische Kohlenwasserstoffe durchgeführt werden, welche z.T. vorteilhafterweise geeignet sind eine homogene wässrig/organische Phase herbeizuführen.

Als Lösemittel ganz besonders bevorzugt wird dabei mindestens ein Vertreter ausgewählt aus der Gruppe, bestehend aus Methanol, Ethanol, n- oder Isopropanol, tert-, iso- oder n-Butanol, Methoxyethanol, Ethoxyethanol, Ethylenglykol, Propylenglykol, Ethylendiglykol, Propylendiglykol, Alkylglylolether, 1,3- oder 1,4-Dioxan, Tetrahydrofuran, Aceton, Heptan, Cyclohexan, Ethylcyclohexan, Toluol und Xylol, eingesetzt.

Die geeignete Menge des Lösungsmittel ist dabei vom Fachmann z.B. durch einfache Handversuche leicht zu ermitteln.

Die Temperatur, bei der die Reaktion durchgeführt wird, kann in weiten Grenzen variiert werden. Vorzugsweise wird die Reaktion jedoch zwischen 20 °C und 150 °C , insbesondere zwischen 50 °C und 90 °C, aber ganz besonders bevorzugt zwischen 60 °C und 80 °C durchgeführt.

Beim erfindungsgemäßen Verfahren ist es im allgemeinen vorteilhaft, dass das zu oxidierende Amin [I], Alkali- und/oder Ammoniumhydrogencarbonat, die Brönstedtsäuren und ggf. Lösungsmittel vorgelegt werden und das Wasserstoffperoxid dem Reaktionsgemisch zugefügt wird. Dies kann sowohl absatzweise als auch kontinuierlich geschehen, vorzugsweise über einen Zeitraum von 0,1 bis 72 Stunden, insbesondere über einen Zeitraum von 2 bis 40 Stunden, ganz besonders bevorzugt jedoch von 4 bis 10 Stunden.

Ebensogut können aber auch alle der vorstehend genannten Komponenten gleichzeitig im jeweils gewünschten Verhältnis kontinuierlich einem Reaktorsystem zugeführt werden. Das erfindungsgemäße Verfahren kann also sowohl diskontinuierlich, als auch besonders vorteilhaft teilkontinuierlich oder kontinuierlich durchgeführt werden.

Bei der kontinuierlichen bzw. teilkontinuierlichen Ausführungsform können alle dafür dem Fachmann bekannten Reaktorsysteme insbesondere Rohrreaktoren, Reaktorkaskaden mit mindestens 2 Reaktoren, die über ein Rühr- bzw. Mischorgan verfügen können, oder auch Kombinationen aus beiden verwendet werden.

Ein besonderer Vorteil des erfindungsgemäßen Verfahrens ist es, dass die Reaktion unter Bedingungen durchgeführt werden kann, bei denen das Reaktionsmedium permanent oder auch nur zeitweise mit Metalloberflächen in Kontakt treten kann, ohne dass dabei große Verluste an Wasserstoffperoxid oder Einbußen an Umsatz bzw. Selektivität in Kauf genommen werden müssten. Dies ist auch unter Gesichtspunkten der Verfahrenssicherheit von besonderer Bedeutung, da eine unkontrollierte Zersetzung von H₂O₂ gerade bei einem industriellen Verfahren fatale Folgen haben kann.

Die erfindungsgemäße Reaktion wird daher besonders bevorzugt in Gegenwart von Metalloberflächen, welche Legierungen aus Eisen, Titan, Zirkon, Vanadium, Niob, Tantal, Chrom, Molybdän, Wolfram, Mangan, Cobalt, Nickel, Kupfer, Zink, und/oder Aluminium enthalten, wie sie zum Bau von Chemiereaktoren üblicherweise eingesetzt werden, durchgeführt, so dass keine in speziellen Werkstoffen ausgelegten Apparaturen und auch keine besonderen Vorbehandlung der Apparaturen vonnöten sind, was einen ganz besonderen Vorteil des Verfahrens darstellt.

Das erfindungsgemäße Verfahren zeichnet sich ferner durch eine außerordentliche Robustheit und Unempfindlichkeit gegen Störfaktoren aus. Neben vorteilhaften geringeren H₂O₂-Verbrauch gewährleistet das Verfahren insgesamt einen deutlichen Gewinn an Sicherheit.

Darüber hinaus wurde nun überraschenderweise gefunden, dass man im Falle von Dihydrogenphosphat auf die Verwendung von Hydrogencarbonat als Katalysator ganz verzichten kann und lediglich unter Zusatz von Alkali- und/oder Ammoniumdihydrogenphosphat als Katalysator noch vergleichsweise gute Ausbeuten an 4-substituierten 2,2,6,6-Tetramethyl-piperidin-N-oxy-verbindungen [II] oder Gemischen aus [II] und 4-substituierten 2,2,6,6-Tetramethyl-piperidin-N-hydroxy-verbindungen [III] erhält. Dies kann von besonderem Vorteil sein, weil dabei das katalytisch wirkende Hydrogencarbonat komplett eingespart werden kann.
Die Reaktion kann dabei auch wahlweise in Gegenwart mindestens einem der o.g. Lösungsmittel durchgeführt werden.

Gegenstand der vorliegenden Erfindung ist somit auch ein Verfahren zur Herstellung von 4-substituierten 2,2,6,6-Tetramethyl-piperidin-N-oxy-verbindungen [II] oder von Gemischen aus [II] und 4-substituierten 2,2,6,6-Tetramethyl-piperidin-N-hydroxy-verbindungen [III] 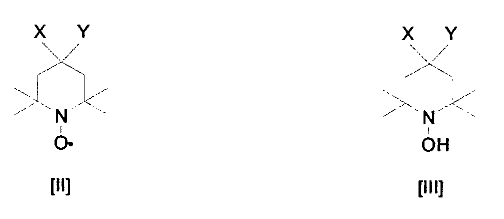 wobei X+Y = O sein kann oder für ein cyclisches Ketal stehen kann mit den Resten 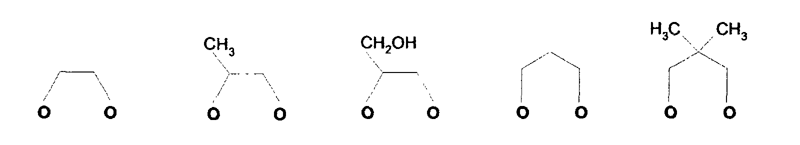 oder für ein offenkettiges Ketal stehen kann, bei dem X= O-R und Y= O-R' bedeuten, wobei R und R' gleich oder verschieden voneinander sein können und jeweils für CH₃, CH₂-CH₃, CH₂-CH₂-CH₃, CH(CH₃)-CH₃, CH₂-CH₂-CH₂-CH₃ und CH₂-CH(CH₃)-CH₃ stehen können,
durch Oxidation von entsprechenden 4-substituierten 2,2,6,6-Tetramethylpiperidinen [I] 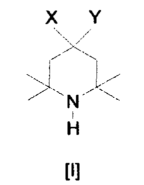 mit Hilfe von Wasserstoffperoxid ohne Gegenwart von Hydrogencarbonat, bei dem die Reaktion, ggf. in Gegenwart eines Lösungsmittels, unter Zusatz von Alkali- und/oder Ammoniumdihydrogenphosphat als Katalysator durchgeführt wird.
Dabei wird die Reaktion vorzugsweise so gefahren, dass die wässrige Phase im Falle eines zweiphasigen Gemisches bzw. die wässrig/organische Phase im Falle einer homogenen Reaktionsmediums nach Beendigung der Reaktion einen pH-Wert von 7,5 bis 9,0 aufweist.

Die nachfolgenden Beispiele dienen zur Erläuterung der Erfindung, ohne dass hierdurch eine eine Beschränkung erfolgen soll.

### Beispiel 1 (nicht erfindungsgemäß)

In einem ummantelten 1 L-Glasreaktor mit einem Glasflügelrührer wurden 9,5 mol vollentsalztes Wasser (VE-Wasser), 0,21 mol NaHCO₃ und 2,0 mol Triacetonamin (TAA) vorgelegt, das Rektionsgemisch unter Rühren (400 U/Min.) mit einem Thermostaten auf eine Temperatur von 60 °C eingestellt und innerhalb von 4 Stunden 3,5 mol Wasserstoffperoxid als 50 %ige wässrige Lösung über einen gekühlten Tropftrichter gleichmäßig zudosiert. Die Reaktionstemperatur wurde bei 57 bis 63 C gehalten. Das beim Prozess anfallende Abgas, das überwiegend aus O₂ bestand, wurde volumetrisch nach dem Verdrängungsprinzip gemessen. Von dem zweiphasigen Reaktionsgemisch wurde nach dem Ende der Zudosierung eine Probe gezogen und die Produktzusammensetzung in der organischen Phase gaschromatographisch analysiert. Von der unteren wässrigen Phase wurde bei Raumtemperatur der pH-Wert gemessen.

### Beispiel 2 (nicht erfindungsgemäß)

Um H₂O₂-zerstörende Bedingungen zu simulieren, wurde an dem Glasflügelrührer eine Stahlgewebepackung angebracht und ansonsten wie in Beispiel 1 verfahren.

### Beispiel 3

Beispiel 3 wurde analog zu Beispiel 2 durchgeführt, jedoch wurden neben der verwendeten Menge von 0,21 mol NaHCO₃ noch 0,06 mol H₃PO₄ als Co-Katalysator eingesetzt.

### Beispiel 4

Beispiel 4 wurde analog zu Beispiel 2 durchgeführt, jedoch wurden neben der verwendeten Menge von 0,21 mol NaHCO₃ noch 0,06 mol H₃PO₄ als Co-Katalysator eingesetzt.

### Beispiel 5

Beispiel 5 wurde analog zu Beispiel 2 durchgeführt, jedoch wurden neben der verwendeten Menge von 0,21 mol NaHCO₃ noch 0,06 mol NaH₂PO₄ *1H₂O als Co-Katalysator eingesetzt.

### Beispiel 6

Beispiel 6 wurde analog zu Beispiel 2 durchgeführt, jedoch wurden neben der verwendeten Menge von 0,21 mol NaHCO₃ noch 0,15 mol NaH₂PO₄ *1H₂O als Co-Katalysator eingesetzt.

### Beispiel 7

Beispiel 7 wurde analog zu Beispiel 2 durchgeführt, jedoch wurden neben der verwendeten Menge von 0,21 mol NaHCO₃ noch 0,05 mol Na₂HPO₄ *2 H₂O als Co-Katalysator eingesetzt.

### Beispiel 8

Beispiel 8 wurde analog zu Beispiel 2 durchgeführt, jedoch wurde als Katalysator ausschließlich NaH₂PO₄*1 H₂O (0,21 mol ) und kein NaHCO₃ verwendet.

### Beispiel 9

Beispiel 9 wurde analog zu Beispiel 2 durchgeführt, jedoch wurden neben der verwendeten Menge von 0,21 mol NaHCO₃ noch 0,06 mol Salpetersäure als Co-Katalysator eingesetzt.

### Beispiel 10

Beispiel 10 wurde analog zu Beispiel 2 durchgeführt, jedoch wurden neben der verwendeten Menge von 0,21 mol NaHCO₃ noch 0,06 mol H₂SO₄ als Co-Katalysator eingesetzt.

### Beispiel 11

Beispiel 11 wurde analog zu Beispiel 2 durchgeführt, jedoch wurden neben der verwendeten Menge von 0,21 mol NaHCO₃ noch 0,06 mol Salzsäure als Co-Katalysator eingesetzt.

### Beispiel 12

Beispiel 12 wurde analog zu Beispiel 2 durchgeführt, jedoch wurden neben der verwendeten Menge von 0,21 mol NaHCO₃ noch 0,06 mol Essigsäure als Co-Katalysator eingesetzt.

### Beispiel 13

Beispiel 13 wurde analog zu Beispiel 2 durchgeführt, jedoch wurden neben der verwendeten Menge von 0,21 mol NaHCO₃ noch 0,06 mol 2-Ethylhexansäure als Co-Katalysator eingesetzt.

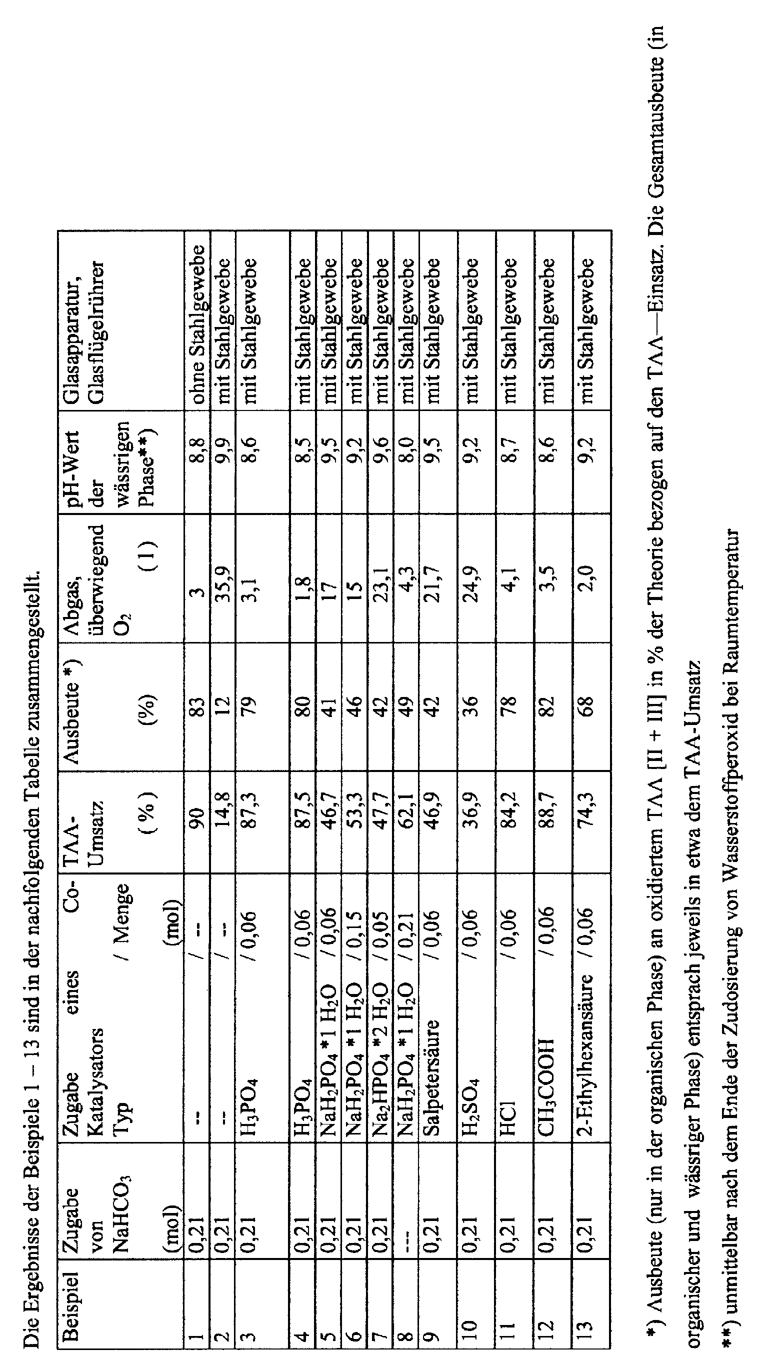

### Beispiel 14 (nicht erfindungsgemäß)

In einem 20 m³ Stahlreaktor wurden 1960 kg VE-Wasser, 2,5 kmol NaHCO₃ und 22,5 kmol TAA vorgelegt, eine Temperatur von 60 °C eingestellt und über 60 Stunden 100 kmol an 50 %iger wässriger H₂O₂-Lösung zudosiert. Das beim Prozess anfallende Abgas wurde durch Zufuhr von N₂ auf eine Sauerstoffkonzentration von < 7 % eingestellt und einer geeigneten Entsorgung (Abgasverbrennung) zugeführt.

### Beispiel 15

Beispiel 15 wurde analog zu Beispiel 14 durchgeführt, jedoch wurde der pH-Wert des Reaktionsgemisches während der Reaktionszeit von 60 Stunden durch Zugabe von H₃PO₄ ( 1 kmol ) auf pH 8 - 9 gehalten.

Die Ergebnisse der Beispiele 14 und 15 sind in der nachfolgenden Tabelle zusammengestellt.

| Beispiel | Zugabe von NaHCO₃ (kmol) | Zugabe eines Typ | Co-Katalysators / Menge (kmol) | TAA-Umsatz (%) | pH-Wert der wässrigen Phase**) | Reaktor |
|---|---|---|---|---|---|---|
| 14 | 2,5 | -- | / -- | 36 | 10,5 | Stahl-reaktor |
| 15 | 2,5 | H₃PO₄ | / 1 | 93 | 8 | Stahl-reaktor |

| | | | | | | |
|---|---|---|---|---|---|---|
| **) unmittelbar nach dem Ende der Zudosierung von Wasserstoffperoxid bei Raumtemperatur | | | | | | |

### Beispiel 16 (nicht erfindungsgemäß)

In einem ummantelten 1 L-Glasreaktor mit einem Glasflügelrührer wurden 11,7 mol VE-Wasser, 0,21 mol NaHCO₃ und 2,0 mol Triacetonamin-Ethylenglykol-Ketal (TAA-EGK) vorgelegt, das Reaktionsgemisch unter Rühren (400 U/Min.) mit einem Thermostaten auf 60 °C eingestellt und innerhalb von 4 Stunden 2,8 mol Wasserstoffperoxid als 50 %ige wässrige Lösung über einen gekühlten Tropftrichter gleichmäßig zudosiert. Die Reaktortemperatur wurde bei 57 bis 63 °C gehalten. Das beim Prozess anfallende Abgas, das überwiegend aus O₂ bestand wurde volumetrisch nach dem Verdrängungsprinzip gemessen.

Von dem zweiphasigen Reaktionsgemisch wurde nach dem Ende der Zudosierung eine Probe gezogen und die Produktzusammensetzung in der organischen Phase gaschromatographisch analysiert. In der unteren wässrigen Phase wurde bei Raumtemperatur der pH-Wert gemessen.

### Beispiel 17

Um H₂O₂-zerstörende Bedingungen zu simulieren wurde an dem Glasflügelrührer eine Stahlgewebepackung angebracht und ansonsten wie in Beispiel 16 verfahren.

### Beispiel 18

Beispiel 18 wurde analog zu Beispiel 17 durchgeführt, jedoch wurden neben der verwendeten Menge von 0,21 mol NaHCO₃ noch 0,06 mol H₃PO₄ als Co-Katalysator eingesetzt.

Die Ergebnisse der Beispiele 16 - 18 sind in der nachfolgenden Tabelle zusammengestellt.

| Beispiel | Zugabe von NaHCO₃ (mol) | Zugabe eines Co-Katalysators Typ / Menge / (mol) | | TAAEGK- Umsatz (%) | Ausbeute*)(%) | Abgas, über- wiegend O₂ (1) | pH-Wert der wässrigen Phase nach der Reaktion | Glasapparatur, Glasflügel- rührer |
|---|---|---|---|---|---|---|---|---|
| 16 | 0,21 | -- | / -- | 82 | 77 | 6 | 9,2 | ohne Stahlgewebe |
| 17 | 0,21 | -- | / -- | 39 | 38 | 22 | 9,7 | mit Stahlgewebe |
| 18 | 0,21 | H₃PO₄ | / 0,06 | 75 | 70 | 5,6 | 9,2 | mit Stahlgewebe |

| | | | | | | | | |
|---|---|---|---|---|---|---|---|---|
| *) Ausbeute an oxidiertem TAA-EGK [II + III] in % der Theorie (nur die organische Phase) bezogen auf den TAA-EGK-Einsatz. Die Gesamtausbeute (in organischer und wässriger Phase) entsprach jeweils in etwa dem TAA- EGK-Umsatz | | | | | | | | |

### Beispiel 19 (kontinuierlich)

Zwei in Reihe geschaltete Glasreaktoren (A1, A2) mit Glasflügelrührern wurden jeweils bis zum Überlauf mit 0,46 l eines Reaktionsgemisches entsprechend dem Beispiel 18 befüllt, auf 60 °C eingestellt und über Dosierpumpen folgende Eduktströme zudosiert:

| In Reaktor: | A1 | A2 |
|---|---|---|
| Mengen in | (mol/h) | (mol/h) |
| wässrige NaHCO₃-Lösung ( 7,8 %ig ) | 0,029 | --- |
| TAA-EGK: | 0,26 | --- |
| wässrige H₂O₂-Lösung ( 50 %ig ): | 0,40 | 0,10 |
| wässrige H₃PO₄ ( 85 %ig ): | 0,028 | 0,007 |

Aus den Mengenangaben ergibt sich eine mittlere Verweilzeit pro Reaktor von 4 Stunden.

Die Reaktionsprodukte gelangten per Überlauf aus dem Reaktor A1 in den Reaktor A2 und von dort per Überlauf in eine Glasvorlage. Das beim Prozess anfallende Abgas ( überwiegend O₂) wurde über eine Gassammelleitung ausgetragen und die Abgasmenge volumetrisch nach dem Verdrängungsprinzip bestimmt.

Nach Erreichen des Gleichgewichtszustandes wurden aus den zweiphasigen Reaktionsgemischen in den Reaktoren bzw. der Glasvorlage Proben gezogen. In der unteren wässrigen Phase aus der Glasvorlage wurde zunächst der pH-Wert bei Raumtemperatur gemessen. Alle Proben wurden mit einem alkoholischen Lösemittel verdünnt und die Produktzusammensetzung des danach einphasigen Produktgemisches jeweils gaschromatographisch analysiert:

| Reaktor: | A1 | A2 | Glasvorlage |
|---|---|---|---|
| TAA-EGK-Umsatz ( % ): | 79 | 88 | 89 |
| Ausbeute(%)^{*)}: | 78 | 87 | 88 |
| pH-Wert der wässrigen Phase: | | | 7,7 |
| Abgasmenge(l/h): | | | 1,2 |

| | | | |
|---|---|---|---|
| *) Ausbeute an oxidiertem TAA-EGK [II + III] in %der Theorie bezogen auf den TAA-EGK-Einsatz | | | |

## Patentansprüche

1. Verfahren zur Herstellung von 4-substituierten 2,2,6,6-Tetramethyl-piperidin-N-oxy-verbindungen [II] oder von Gemischen aus [II] und 4-substituierten 2,2,6,6-Tetramethyl-piperidin-N-hydroxy-verbindungen [III] 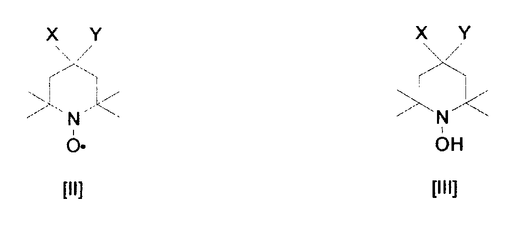 wobei X+Y = O sein kann oder für ein cyclisches Ketal stehen kann mit den Resten 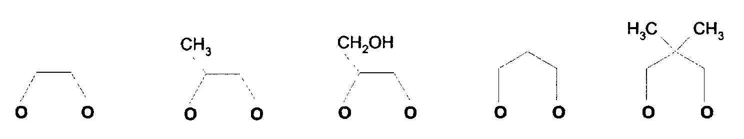 oder für ein offenkettiges Ketal stehen kann, bei dem X= O-R und Y= O-R' bedeuten, wobei R und R' gleich oder verschieden voneinender sein können und jeweils für CH₃, CH₂-CH₃, CH₂-CH₂-CH₃, CH(CH₃)-CH₃, CH₂-CH₂-CH₂-CH₃ und CH₂-CH(CH₃)-CH₃ stehen können,
durch Oxidation von entsprechenden 4-substituierten 2,2,6,6-Tetramethylpiperidinen [I] mit Hilfe von Wasserstoffperoxid in Gegenwart von Alkali- und/oder 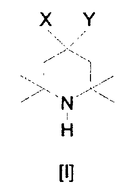 Ammoniumhydrogencarbonat und ggf. in Gegenwart eines Lösungsmittels,
**dadurch gekennzeichnet,**
**dass** die Reaktion unter Zusatz von solchen Brönstedtsäuren durchgeführt wird, die eine Säurestärke größer als die des eingesetzten Hydrogencarbonats besitzen.

2. Verfahren nach Anspruch 1,
**dadurch gekennzeichnet,**
**dass** als Alkalihydrogencarbonat, Lithium-, Natrium-, Kalium-, Rubidium- und/oder Cäsiumhydrogencarbonat eingesetzt werden.

3. Verfahren nach Anspruch 1 oder 2,
**dadurch gekennzeichnet,**
**dass** 0,02 bis 0,5 Moleq Alkali- und/oder Ammoniumhydrogencarbonat bezogen auf das zu oxidierende Amin [I] eingesetzt werden.

4. Verfahren nach einem der vorhergehenden Ansprüche,
**dadurch gekennzeichnet,**
**dass** als Brönstedtsäuren eine oder mehrere anorganische Säuren eingesetzt werden.

5. Verfahren nach einem der Ansprüche 1 bis 3,
**dadurch gekennzeichnet,**
**dass** als Brönstedtsäuren eine oder mehrere organische Säuren eingesetzt werden

6. Verfahren nach einem der Ansprüche 1 bis 3,
**dadurch gekennzeichnet,**
**dass** als Brönstedtsäuren eine Mischung aus einer oder mehreren anorganischen Säuren und aus einer oder mehreren organischen Säuren eingesetzt wird.

7. Verfahren nach Anspruch 4 oder nach Anspruch 6,
**dadurch gekennzeichnet,**
**dass** als anorganische Säure Phosphorsäure, Dihydrogenphosphat, Hydrogenphosphat, Salpetersäure, Schwefelsäure, Hydrogensulfat und/oder Halogenwasserstoffsäuren eingesetzt werden.

8. Verfahren nach Anspruch 5 oder nach Anspruch 6,
**dadurch gekennzeichnet,**
**dass** als organische Säure Ameisensäure oder eine gesättigte lineare oder ggf. verzweigte ein- oder mehrbasige aliphatische Carbonsäure mit 2 bis 12 C-Atomen verwendet wird, die mit O-R¹ oder NR²R³ substituiert sein kann, wobei R¹, R² bzw. R³ gleich oder verschieden sein können und Wasserstoff, einen aliphatischen oder cycloaliphatischen, gesättigten Alkylrest mit 1 bis 12 C-Atomen oder eine Carboxyalkylgruppe (CH₂)ₙCOOH mit n = 1 bis 5 bedeuten oder R² und R³ beide zusammen für eine gesättigte, gegebenenfalls alkylsubstituierte Alkylenkette mit 4 bis 11 C-Atomen stehen können, mit der Maßgabe, dass wenn R² und R³ gleichzeitig Wasserstoff oder Alkyl oder beide zusammen Alkylen bedeuten oder wenn R² = Wasserstoff und R³ = Alkyl bedeuten, die mit NR²R³ substituierte Carbonsäure mehrbasig sein muß.

9. Verfahren nach Anspruch 8,
**dadurch gekennzeichnet,**
**dass** Essigsäure, Hydroxyessigsäure, Methoxyessigsäure, Propionsäure, Buttersäure, 2-Ethylhexansäure, Oxalsäure, Malonsäure, Bernsteinsäure, Glutarsäure, Zitronensäure, Iminodiessigsäure, Nitrilotriessigsäure oder eine saure Aminosäure eingesetzt werden.

10. Verfahren nach Anspruch 5 oder nach Anspruch 6,
**dadurch gekennzeichnet,**
**dass** als organische Säuren Phosphonsäuren und/oder ihre partiellen Salze mit der folgenden allgemeinen Formel:
Z(PO₃HₙM₂₋ₙ)ₘ
einzeln oder auch als Gemisch eingesetzt werden, wobei Z ein oder mehrere, lineare oder verzweigte alkylische Radikale oder Diradikale mit insgesamt C₁-C₁₀ darstellt, die insgesamt bis zu 3 Stickstoffatome enthalten können, m Werte von 1 bis 6 und n Werte von 1 oder 2 annehmen kann und M = Alkali oder NH₄ ist.

11. Verfahren nach einem der vorhergehenden Ansprüche,
**dadurch gekennzeichnet,**
**dass** man bezogen auf das zu oxidierende Amin [I] 0,01 - 0,5 Moleq, vorzugsweise jedoch 0,03-0,1 Moleq der Brönstedtsäuren einsetzt.

12. Verfahren nach einem der vorhergehenden Ansprüche,
**dadurch gekennzeichnet,**
**dass** die Reaktion so durchgeführt wird, dass die wässrige Phase im Falle eines zweiphasigen Gemisches bzw. die wässrig/organische Phase im Falle eines homogenen Reaktionsmediums nach Beendigung der Reaktion einen pH-Wert von 7,0 bis 10,0 aufweist.

13. Verfahren nach Anspruch 12,
**dadurch gekennzeichnet,**
**dass** die Reaktion so durchgeführt wird, dass die wässrige Phase im Falle eines zweiphasigen Gemisches bzw. die wässrig/organische Phase im Falle eines homogenen Reaktionsmediums nach Beendigung der Reaktion einen pH-Wert von 8,0 bis 9,6, vorzugsweise von 8,2 bis 9,2 aufweist.

14. Verfahren nach einem der vorhergehenden Ansprüche,
**dadurch gekennzeichnet,**
**dass** Wasserstoffperoxid in wässriger Lösung mit einer Konzentration im Bereich von 10 bis 90 Gew.%, vorzugsweise im Bereich von 20 bis 60 Gew.%, ganz besonders bevorzugt von 30 bis 50 Gew.%verwendet wird.

15. Verfahren nach einem der vorhergehenden Ansprüche,
**dadurch gekennzeichnet,**
**dass** als Lösemittel Alkohole, Diole, Etherverbindungen, Ketone, aliphatische, cycloaliphatische, aromatische und/oder araliphatische Kohlenwasserstoffe eingesetzt werden.

16. Verfahren nach Anspruch 15,
**dadurch gekennzeichnet,**
**dass** als Lösemittel mindestens ein Vertreter ausgewählt aus der Gruppe, bestehend aus Methanol, Ethanol, n- oder Isopropanol, tert-, iso- oder n-Butanol, Methoxyethanol, Ethoxyethanol, Ethylenglykol, Propylenglykol, Ethylendiglykol, Propylendiglykol, Alkylglykolether, 1,3- oder 1,4-Dioxan, Tetrahydrofuran, Aceton, Heptan, Cyclohexan, Ethylcyclohexan, Toluol und Xylol, eingesetzt wird.

17. Verfahren nach einem der vorhergehenden Ansprüche,
**dadurch gekennzeichnet,**
**dass** die Reaktion zwischen 20 °C und 150 °C, vorzugsweise zwischen 50 °C und 90 °C, insbesondere zwischen 60 °C und 80 °C durchgeführt wird.

18. Verfahren nach einem vorhergehenden Ansprüche,
**dadurch gekennzeichnet,**
**dass** das zu oxidierende Amin [I], Alkali- und/oder Ammoniumhydrogencarbonat, die Brönstedtsäuren und ggf. Lösungsmittel vorgelegt werden und das Wasserstoffperoxid dem Reaktionsgemisch absatzweise oder kontinuierlich über einen Zeitraum von 0,1 bis 72 Stunden zugegeben wird.

19. Verfahren nach Anspruch 18,
**dadurch gekennzeichnet,**
**dass** die Dauer der Zugabe 2 bis 40 Stunden, vorzugsweise 4 bis 10 Stunden beträgt.

20. Verfahren nach einem der vorhergehenden Ansprüche,
**dadurch gekennzeichnet,**
**dass** alle Komponenten im jeweils gewünschten Verhältnis gleichzeitig und kontinuierlich einem Reaktorsystem zugeführt werden.

21. Verfahren nach Anspruch 20,
**dadurch gekennzeichnet,**
**dass** das Reaktorsystem aus einem Rohrreaktor oder einer Reaktorkaskade mit mindestens 2 Reaktoren oder einer Kombination aus beiden besteht.

22. Verfahren nach einem der vorhergehenden Ansprüche,
**dadurch gekennzeichnet,**
**dass** die Reaktion unter Bedingungen durchgeführt wird, bei denen das Reaktionsmedium wenigstens zeitweise mit Metalloberflächen in Kontakt tritt.

23. Verfahren nach Anspruch 22,
**dadurch gekennzeichnet,**
**dass** die Metalloberflächen Legierungen aus Eisen, Titan, Zirkon, Vanadium, Niob, Tantal, Chrom, Molybdän, Wolfram, Mangan, Cobalt, Nickel, Kupfer, Zink, und/oder Aluminium enthalten, wie sie zum Bau von Chemiereaktoren üblicherweise eingesetzt werden.

24. Verfahren zur Herstellung von 4-substituierten 2,2,6,6-Tetramethyl-piperidin-N-oxy-verbindungen [II] oder von Gemischen aus [II] und 4-substituierten 2,2,6,6-Tetramethyl-piperidin-N-hydroxy-verbindungen [III] 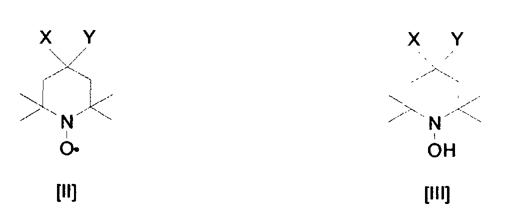 wobei X+Y = O sein kann oder für ein cyclisches Ketal stehen kann mit den Resten 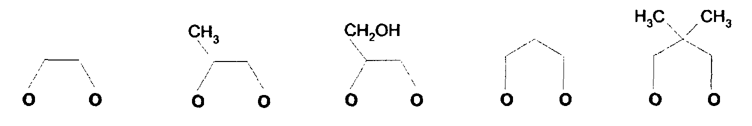 oder für ein offenkettiges Ketal stehen kann, bei dem X= O-R und Y= O-R' bedeuten, wobei R und R' gleich oder verschieden voneinander sein können und jeweils für CH₃, CH₂-CH₃, CH₂-CH₂-CH₃, CH(CH₃)-CH₃, CH₂-CH₂-CH₂-CH₃ und CH₂-CH(CH₃)-CH₃ stehen können,
durch Oxidation von entsprechenden 4-substituierten 2,2,6,6-Tetramethylpiperidinen [I] 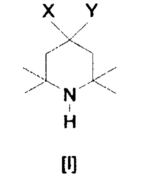 mit Hilfe von Wasserstoffperoxid ohne Gegenwart von Hydrogencarbonat,
**dadurch gekennzeichnet,**
**dass** die Reaktion, ggf. in Gegenwart eines Lösungsmittels, unter Zusatz von Alkali-und/oder Ammoniumdihydrogenphosphat als Katalysator durchgeführt wird.
